# EUROPEAN PATENT APPLICATION

(11) **EP 4 129 387 A1**
(43) Date of publication of application: **08.02.2023**
(21) Application number: 21780246.1
(22) Date of filing: 31.03.2021
(51) Int. Cl.: A61M 25/10, A61B 18/04

(54) **BALLOON CATHETER AND BALLOON CATHETER SYSTEM**

(30) Priority: 31.03.2020 JP 2020064405
(71) Applicant: Toray Industries, Inc., Tokyo 103-8666 (JP)
(72) Inventor: WATANABE Akio, Otsu-shi, Shiga 520-2141 (JP); TSUKAMOTO Kota, Tokyo 103-8666 (JP)
(74) Representative: Kador & Partner PartG mbB
(86) International application number: PCT/JP2021/013784
(87) International publication number: WO 2021/201082

(57) **Abstract**

A balloon catheter (15) includes: a balloon (25); an outside shaft (30) connected to a proximal end of the balloon; an inside shaft (35) extending inside the outside shaft and into the balloon to be connected to a distal end of the balloon; and a heating member (40) disposed in the balloon for heating a liquid in the balloon. A distance between a distal end (30a) of the outside shaft and a proximal end (40b) of the heating member is between 0 mm and 5 mm.

## Description

### Technical Field

The present invention relates to a balloon catheter and a balloon catheter system.

### Background Art

A catheter ablation therapy is a treatment method that uses a catheter inserted into a body to ablate a target site in the human body. As an example, a target site is destroyed by ablation to treat diseases such as arrhythmia due to atrial fibrillation, endometriosis, cancer, etc. As disclosed in JP3611799B2 and JP4747141B2, a balloon catheter having a balloon at its distal end is known as a catheter used for catheter ablation therapy.

When a balloon catheter is inserted into a body, its balloon is deflated and stretched in a longitudinal direction of the balloon catheter. Then, a liquid is supplied to the balloon catheter inserted in the body so that the balloon is inflated. Since a temperature of the liquid in the balloon is controlled, a surface temperature of the balloon can be controlled. By bringing the balloon whose surface temperature has been controlled to a target one into contact with a circumferential target site, e.g., a connection area of a vein to an atrium, the circumferential target site can be ablated at once.

The temperature of the liquid in the balloon is controlled by heating the liquid in the balloon with a heating member disposed in the balloon. Simply heating of the liquid in the balloon with the heating member cannot raise a surface temperature of the balloon to a target surface temperature, because a temperature gradient in the liquid in the balloon is generated around the heating member. Also, it is difficult to control power to the heating member in such a manner that the surface temperature of the balloon reaches a target surface temperature. For this reason, when the liquid in the balloon is heated with the heating member, the liquid in the balloon is agitated to diffuse the heated liquid in the balloon. However, unless the heated liquid is diffused efficiently, the temperature gradient cannot be reduced, and the surface temperature of the balloon cannot be raised to a target surface temperature. Also, it is still difficult to control power to the heating member in such a manner that the surface temperature of the balloon reaches a target surface temperature. In addition, when diffusion efficiency of the heated liquid is unstable, the surface temperature of the balloon cannot be controlled in a reliable manner.

### Disclosure of the Invention

The present invention has been made in view of the above circumstances. An object of the present invention is to provide a balloon catheter capable of efficiently diffusing a liquid heated in a balloon in a reliable manner, and a catheter system having such a balloon catheter.

A balloon catheter of the present invention comprises: a balloon; an outside shaft connected to a proximal end of the balloon; an inside shaft extending inside the outside shaft and into the balloon to be connected to a distal end of the balloon; and a heating member disposed in the balloon for heating a liquid in the balloon, wherein a distance between a distal end of the outside shaft and a proximal end of the heating member is between 0 mm and 5 mm.

In the balloon catheter of the present invention, a midpoint of the heating member in a direction of a center axis of the inside shaft may be positioned closer to the proximal end of the balloon than a midpoint between the proximal end of the balloon and the distal end thereof.

In the balloon catheter of the present invention, the outside shaft may extend into the balloon.

Alternatively, a balloon catheter of the present invention comprises: a balloon; an outside shaft connected to a proximal end of the balloon; an inside shaft extending inside the outside shaft and into the balloon to be connected to a distal end of the balloon; and a heating member disposed in the balloon for heating a liquid in the balloon; wherein the outside shaft extends into the balloon.

In the balloon catheter of the present invention, a through hole may be made in a part of the outside shaft, the part extending in the balloon.

The balloon catheter of the present invention may further comprise a reinforcement tube covering an outer circumferential surface of the inside shaft, wherein the reinforcement tube may extend over from between a proximal end of the heating member and a distal end of the outside shaft, into the outside shaft.

In the balloon catheter of the present invention, a distance between the proximal end of the heating member and a distal end of the reinforcement tube may be between 0 mm and 5 mm.

The balloon catheter of the present invention may comprise a temperature sensor disposed in an inner space of the balloon.

A balloon catheter system of the present invention comprises: the aforementioned balloon catheter; a supply device that supplies a liquid into a liquid delivery path formed between the outside shaft and the inside shaft; an agitation device that agitates a liquid in the balloon by repeating supply of liquid to the liquid delivery path and discharge of liquid from the liquid delivery path; and a heating device electrically connected to the heating member to supply electric energy to the heating member.

Alternatively, a balloon catheter system of the present invention comprise: the aforementioned balloon catheter; a supply device that supplies a liquid into a liquid delivery path formed between the outside shaft and the inside shaft; an agitation device that agitates a liquid in the balloon by repeating supply of liquid to the liquid delivery path and discharge of liquid from the liquid delivery path; and a heating device electrically connected to the heating member to supply electric energy to the heating member, wherein the heating device supplies electric energy to the heating member based on information on a temperature acquired by the temperature sensor.

The present invention enables a liquid heated in a balloon to be efficiently diffused in a reliable manner.

### Brief Description of The Drawings

Fig. 1 is a view for describing a first embodiment, showing a balloon catheter system and a balloon catheter.
Fig. 2 is a view showing a distal end portion of the balloon catheter of Fig. 1, with a balloon being inflated.
Fig. 3 is a view showing the distal end portion of the balloon catheter of Fig. 1, with the balloon being deflated and stretched.
Fig. 4 is a cross-sectional view of the balloon catheter shown in Fig. 2 along a IV-IV line thereof.
Fig. 5 is a view for describing a method of venting air through a through hole.
Fig. 6 is a view corresponding to Fig. 2, showing a distal end portion of a balloon catheter according to a second embodiment.
Fig. 7 is a view showing a distal end portion of a balloon catheter according to a third embodiment.
Fig. 8 is a cross-sectional view of the balloon catheter shown in Fig. 7 along a VIII-VIII line thereof.
Fig. 9 is a view corresponding to Fig. 2, showing the distal end portion of the balloon catheter shown in Fig. 7.
Fig. 10 is a view corresponding to Fig. 3, showing the distal end portion of the balloon catheter shown in Fig. 9, with a balloon being deflated and stretched.
Fig. 11 is a view for describing an experiment method using the balloon catheter system.
Fig. 12 is a view showing a conventional balloon catheter which is pressed against an ostia venarum pulmonalium.
Fig. 13 is a view showing a conventional balloon catheter which is pressed against an ostia venarum pulmonalium.

### Mode for Carrying out the Invention

### <First Embodiment>

A first embodiment of the present invention will be described hereunder with reference to specific examples shown in the drawings. In the drawings attached to the specification, a scale dimension, an aspect ratio and so on are changed and exaggerated from the actual ones, for the convenience of easiness in illustration and understanding. In addition, terms used herein to specify shapes, geometric conditions and their degrees, e.g., "parallel", "orthogonal", "same", etc., and values of a length and an angle are not limited to their strict definitions, but construed to include a range capable of exerting a similar function.

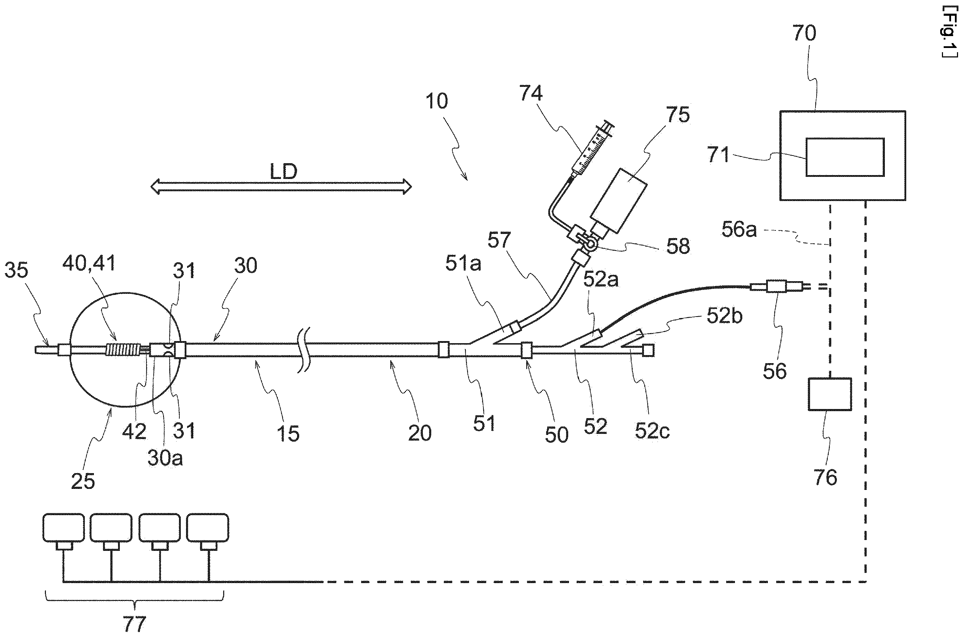

A balloon catheter system 10 shown in Fig. 1 has a balloon catheter 15, and a heating device 70, a supply device 74 and an agitation device 75 connected to the balloon catheter 15. The balloon catheter 15 also has a catheter body 20 having a longitudinal direction LD, and a handle 50 connected to a proximal end of the catheter body 20.

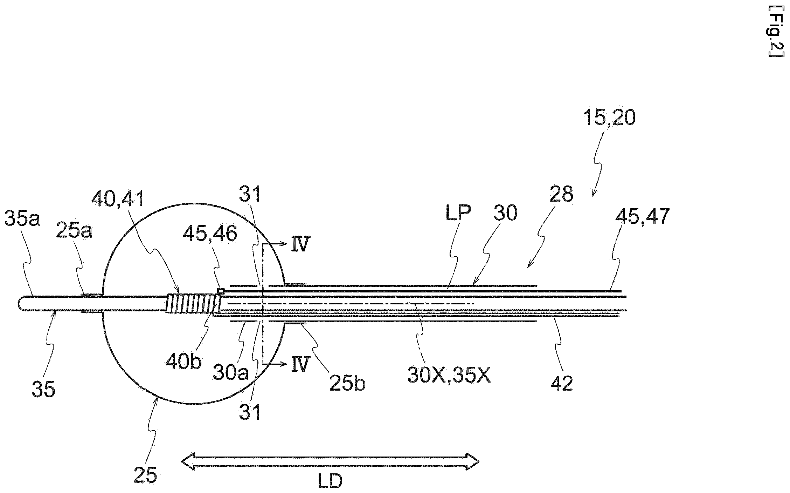

As shown in Fig. 2, the catheter body 20 according to this embodiment has a balloon 25, an outside shaft 30 connected to a proximal end 25b of the balloon 25, an inside shaft 35 connected to a distal end 25a of the balloon 25, and a heating member 40 disposed in the balloon 25. The inside shaft 35 extends inside the outside shaft 30 and into the balloon 25. A liquid delivery path LP in communication with an inside of the balloon 25 is formed between the outside shaft 30 and the inside shaft 35. The heating member 40 is for heating a liquid in the balloon 25.

The longitudinal direction LD of the catheter body 20 is specified as a direction along which center axes 30X, 35X of the outside shaft 30 and the inside shaft 35 extending from the outside shaft 30 extend. In this specification, the term "distal" side used for respective components of the balloon catheter 15 and the catheter body 20 means a side farther from an operator (surgeon) operating the handle 50 and the balloon catheter 15 along the longitudinal direction LD of the catheter body 20, in other words, a distant side. In addition, the term "proximal" side used for respective components of the balloon catheter 15 and the catheter body 20 means a side closer to the operator (surgeon) operating the handle 50 and the balloon catheter 15 along the longitudinal direction LD of the catheter body 20, in other words, a near side.

The balloon catheter system 10 and the balloon catheter 15 are further described in detail hereunder. First, the catheter body 20 of the balloon catheter 15 is described in detail. As described above, the catheter body 20 of the balloon catheter 15 according to this embodiment has the balloon 25, the outside shaft 30, the inside shaft 35, and the heating member 40. Further, the catheter body 20 of this embodiment has a temperature sensor 45 disposed in an inner space of the balloon 25.

The outside shaft 30 and the inside shaft 35 both have a tubular shape, typically a cylindrical shape. Thus, each of the outside shaft 30 and the inside shaft 35 forms a lumen as its inner space. A not-shown guide wire, for example, is inserted through the lumen formed by the inside shaft 35. The inside shaft 35 is inserted through the lumen formed by the outside shaft 30. Namely, the outside shaft 30 and the inside shaft 35 form a dual shaft structure. An internal diameter of the outside shaft 30 is larger than an external diameter of the inside shaft 35. Thus, a lumen remains between the outside shaft 30 and the inside shaft 35. The lumen between the outside shaft 30 and the inside shaft 35 forms the liquid delivery path LP. As shown in Fig. 2, the liquid delivery path LP is in communication with the inside of the balloon 25. The liquid delivery path LP extends into the handle 50.

Lengths of the outside shaft 30 and the inside shaft 35 are preferably between 500 mm or more and 1700 mm or less, and more preferably between 600 mm or more and 1200 mm or less. The outside shaft 30 and the inside shaft 35 are preferably made of a flexible material with excellent antithrombotic properties. The flexible material with excellent antithrombotic properties may include, for example, fluoropolymers, polyamides, polyurethane-based polymers, or polyimides, but is not limited thereto. The outside shaft 30 is preferably manufactured by stacking layers of different flexible materials, in order to have both slidability to the inside shaft 35 and adhesion or heat weldability to the balloon 25.

The external diameter of the outside shaft 30 is preferably between 3.0 mm or more and 4.0 mm or less. The internal diameter of the outside shaft 30 is preferably between 2.5 mm or more and 3.5 mm or less. The external diameter of the inside shaft 35 is preferably between 1.4 mm or more and 1.7 mm or less. The internal diameter of the inside shaft 35 is preferably between 1.0 mm or more and 1.5 mm or less.

The balloon 25 is connected to the outside shaft 30 and the inside shaft 35. The balloon 25 is inflatable when it is filled with a liquid and deflatable when the liquid is discharged therefrom. The balloon 25 is preferably shaped to fit a target site to be treated (e.g., blood vessel). As an example, the balloon 25 adapted for a pulmonary vein junction of a left atrium may have a spherical shape having a diameter between 15 mm or more and 40 mm or less. The spherical shape here includes a true spherical shape, a prolate shape, and a prolate spheroid shape. It also includes a substantially spherical shape.

When an electrode for high-frequency current conduction 41 is used as the heating member 40 as described below, a film thickness of the balloon 25 is preferably thinner, in order that its impedance is lower at a frequency of electric energy supplied by the heating device 70 serving as means for supplying high-frequency power. However, if the balloon 25 is excessively thin, it may cause the balloon 25 a puncture when a surface of the balloon 25 is damaged. Thus, the thickness of the balloon 25 is preferably between 5 µm and 300 µm, and more preferably 10 µm and 200 µm. Note that the thickness is not limited to the above range because the impedance of the balloon 25 varies greatly depending on a dielectric constant of a material of the balloon 25 and a frequency of the electric energy.

A material of the balloon 25 is preferably an elastic material with excellent antithrombotic properties. Specifically, a polyurethane-based polymer material can be used. The polyurethane-based polymer material applicable to the balloon 25 may be, for example, thermoplastic polyether urethane, polyether polyurethane urea, fluorinated polyether urethane urea, polyether polyurethane urea resin, or polyether polyurethane urea amide.

The distal end (distant end) 25a of the balloon 25 is fixed close to the distal end (distant end) 35a of the inside shaft 35, and the proximal end (near end) 25b of the balloon 25 is fixed close to the distal end (distant end) 30a of the outside shaft 30. The balloon 25 can be connected to the outside shaft 30 and the inside shaft 35 by adhesion or heat welding.

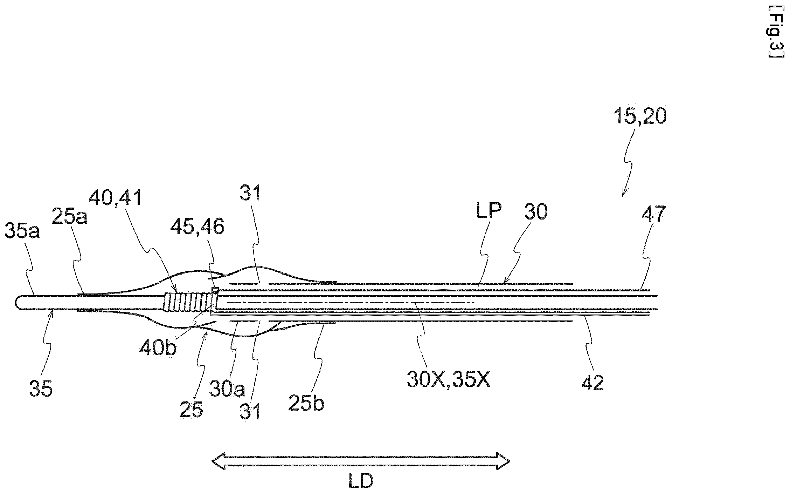

Due to the above fixation of the balloon 25 to the dual tube shaft, the relative movement between the inside shaft 35 and the outside shaft 30 deforms the balloon 25 , and allows to adjust a length of the balloon 25 along the longitudinal direction LD. Specifically, as shown in Fig. 3, when the inside shaft 35 is relatively moved with respect to the outside shaft 30 toward the distal side in the longitudinal direction LD, the balloon 25 is stretched in the longitudinal direction LD and is further strained. In the illustrated example, the movement range of the inside shaft 35 with respect to the outside shaft 30 toward the distal side in the longitudinal direction LD is restricted by the balloon 25. When the inside shaft 35 is relatively moved with respect to the outside shaft 30 from the state shown in Fig. 3 to the proximal side in the longitudinal direction LD, the balloon 25 becomes loosened. By introducing a liquid into the loosened balloon 25, as shown in Fig. 2, the balloon 25 can be inflated. Namely, the relative movement between the outside shaft 30 and the inside shaft 35 allows the dimension of the balloon 25 to be adjusted in the longitudinal direction LD.

Next, the heating member 40 is described. The heating member 40 is disposed on the inside shaft 35 in the balloon 25. The heating member 40 is a member for heating a liquid filled in the balloon 25. As an example, a nichrome wire that generates heat due to electric resistance can be used as the heating member 40. As another example of the heating member 40, as shown in Figs. 2 and 3, the electrode for high-frequency current conduction 41 can be used. By means of high-frequency current conduction (high-frequency energization) to the heating member 40 as the electrode for high-frequency current conduction 41, a high-frequency current flows between the electrode for high-frequency current conduction 41 and a counter electrode 77 (see Fig. 1) disposed outside, so that the liquid positioned between the electrode for high-frequency current conduction 41 and the counter electrode 77 generates Joule heat. The counter electrode 77 is located on the back of a patient, for example.

In the example shown in Figs. 2 and 3, the electrode for high-frequency current conduction 41 is provided on the inside shaft 35 extending in the balloon 25. The electrode for high-frequency current conduction 41 is electrically connected to a lead wire for high-frequency current conduction 42, in order for high-frequency current conduction. The lead wire for high-frequency current conduction 42 extends to the handle 50 in the lumen between the outside shaft 30 and the inside shaft 35 serving as the liquid delivery path LP.

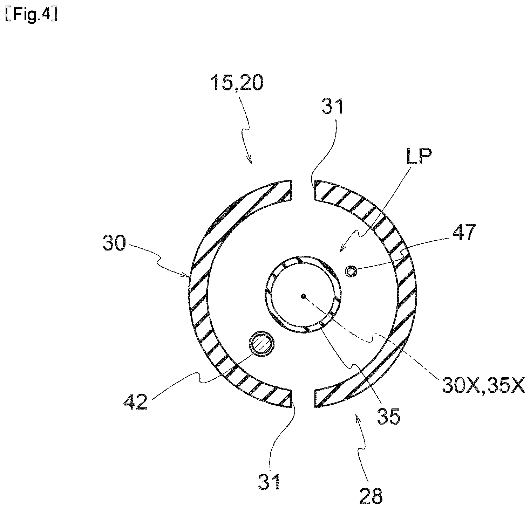

An electrical conductivity of the electrode for high-frequency current conduction 41 is preferably higher than that of the liquid filled in the balloon 25. A conductive material forming the electrode for high-frequency current conduction 41 (and the lead wire for high-frequency current conduction 42) is a metal (e.g., copper, silver, gold, platinum, and alloys thereof), for example. A conductive material other than a metal, e.g., a conductive polymer, can also be used for the electrode for high-frequency current conduction 41 (and the lead wire for high-frequency current conduction 42). When copper is used as a material of the electrode for high-frequency current conduction 41, it is preferable to coat it with a noble metal such as gold, platinum, silver, palladium, etc., to prevent corrosion during long-term use. In addition, a conductive linear part of the lead wire for high-frequency current conduction 42 is preferably coated with an insulating film made of, e.g., fluoropolymer, to prevent short circuit (see Fig. 4).

The electrode for high-frequency current conduction 41 preferably has flexibility in such a manner that the distal end of the balloon catheter 15 can move inside a curved lumen such as a sheath, and can be located at variously positioned cautery sites (e.g., ostia venarum pulmonalium). Thus, the electrode for high-frequency current conduction 41 is preferably formed as a coil electrode formed by winding a metal wire around the inside shaft 35. Alternatively, the electrode for high-frequency current conduction 41 may be formed by winding a flexible tube made of, e.g., highly conductive polymer, around the inside shaft 35.

Diameters of the metal wire forming the electrode for high-frequency current conduction 41 and the lead wire for high-frequency current conduction 42 are preferably between 0.1 mm or more and 1 mm or less, and more preferably between 0.1 mm or more and 0.4 mm or less.

In the illustrated example, as can be understood from Fig. 2, the electrode for high-frequency current conduction 41 is disposed in the middle of the balloon 25 in the direction of the center axis 35X of the inside shaft 35, when the balloon 25 is loosened (or when the balloon 25 is inflated). Specifically, a midpoint of the electrode for high-frequency current conduction 41 in the direction of the center axis 35X of the inside shaft 35 is located at a midpoint between the distal end (distant end) 25a of the balloon 25 and the proximal end (rear end) 25b thereof, when the balloon 25 is loosened (or when the balloon 25 is inflated).

Next, the temperature sensor 45 is described. The temperature sensor 45 is disposed in the inner space of the balloon 25 to acquire information on a temperature of the liquid in the balloon 25. In this embodiment, the temperature sensor 45 has a thermosensitive part 46 located close to the heating member 40. This temperature sensor 45 can acquire information on a temperature of the liquid in the vicinity of the heating member 40. By supplying electric energy to the heating member 40 based on the information acquired by the temperature sensor 45, the liquid around the heating member 40 can be heated to a suitable temperature.

A thermocouple or thermistor can be used as the temperature sensor 45. Information on a temperature, which is acquired by the temperature sensor 45, is an electric potential that can be acquired from the thermocouple, or a resistance value that can be acquired by the thermistor.

As shown in Figs. 2 and 3, the temperature sensor 45 typically has the thermosensitive part 46, and a lead wire for temperature sensor 47 electrically connected to the thermosensitive part 46. When the temperature sensor 45 is a thermocouple, a part where different metals are connected in the thermocouple serves as the thermosensitive part 46. When the temperature sensor 45 is a thermistor, a ceramic element in the thermistor serves as the thermosensitive part 46. The lead wire for temperature sensor 47 is led to the handle 50 in the lumen between the outside shaft 30 and the inside shaft 35 serving as the liquid delivery path LP.

A diameter of the lead wire for temperature sensor 47 is preferably between 0.03 mm or more and 0.5 mm or less, and more preferably between 0.05 mm or more and 0.3 mm or less. When a thermocouple is used as the temperature sensor 45, it may be formed of the lead wire for temperature sensor 47 made of constantan and the lead wire for high-frequency current conduction 42 made of copper, which are welded to each other. In this example, the thermosensitive part 46 made by welding the lead wires 47, 42 can function as a thermocouple. As shown in Fig. 4, the lead wire for temperature sensor 47 is preferably provided with an electrically insulating cover made of, e.g., fluoropolymer or enamel, in order to prevent a short circuit with the lead wire for high-frequency current conduction 42.

Next, the handle 50 connected to the catheter body 20 from the proximal side is described. The handle 50 is a part grasped by an operator (surgeon) during the use of the balloon catheter system 10. Thus, the handle 50 preferably has a design that allows an operator to easily grasp and operate the handle 50 with his/her hand. The handle 50 is preferably made of a material having excellent chemical resistance, such as polycarbonate or ABS resin.

The handle 50 shown in Fig. 1 has a first handle part 51 and a second handle part 52 that are slidable to each other. The first handle part (front handle part) 51 is connected to the outside shaft 30 of the catheter body 20. The second handle part (rear handle part) 52 is connected to the inside shaft 35 of the catheter body 20. By relatively moving the second handle part 52 with respect to the first handle part 51, the inside shaft 35 can be relatively moved with respect to the outside shaft 30.

As shown in Fig. 1, the handle 50 also functions as a part that connects devices included in the balloon catheter system 10 and the balloon catheter 15.

A connector 56 is connected to the second handle part 52. The connector 56 electrically connects the lead wire for high-frequency current conduction 42 and the lead wire for temperature sensor 47 to the external heating device 70. The connector 56 is wired from one branch 52a of a plurality of branches 52a, 52b and 52c provided on the second handle part 52.

The connector 56 preferably has a structure capable of effectively preventing improper connection. In addition, the connector 56 is preferably waterproof. The structure of the connector 56 can be decided in consideration of the surgeon's convenience and as a design matter. Similar to the handle 50, the connector 56 is preferably made of a material having high chemical resistance such as polycarbonate or ABS resin.

The connector 56 may have therein a highly conductive metal pin. The lead wires 42, 47 are connected to this highly conductive metal pin so as to be electrically connectable to the heating device 70 serving as means for supplying high-frequency power. Note that the lead wire for temperature sensor 47 may be electrically connected to a device other than the heating device 70 serving as means for supplying high-frequency power. It may be connected to a temperature indicator, for example. A material of the highly conductive metal pin included in the connector 56 may be of any type, as long as it is a metal having high conductivity. A material of the high conductive metal pin included in the connector 56 may be, for example, copper, silver, gold, platinum, and alloys thereof. In addition, an outside of the highly conductive metal pin is preferably protected by a material having electrically insulating properties and chemical resistance. An electrically insulating and chemically resistant material may be, for example, polysulfone, polyurethane-based polymers, polypropylene, or polyvinyl chloride.

The second handle part 52 has the branches 52b and 52c other than the branch 52a, to which the connector 56 is connected. These branches 52b and 52c serve as a part through which a liquid is supplied to the lumen as an inner space of the inside shaft 35, and a part from which a guide wire inserted through the lumen of the inside shaft 35 extends. During a cardiac ablation therapy, a saline solution the amount of which is as small as about 100 ml/hour is generally injected into a patient's body through the lumen of the inside shaft 35. The injection of saline solution effectively prevents backflow of blood into the lumen of the inside shaft 35.

In addition, as shown in Fig. 1, an extension tube 57 extends from the first handle part 51. The extension tube 57 communicates the liquid delivery path LP of the catheter body 20 with the external supply device 74 or the external agitation device 75. The extension tube 57 extends from the branch 51a provided on the first handle part 51. The extension tube 57 is connected to the supply device 74 and the agitation device 75 via a valve 58. In the illustrated example, whether the supply unit 74 or the agitation device 75 is communicated with the liquid delivery path LP can be selected by operating the valve 58. A three-way stopcock may be used as the valve 58.

Next, devices constituting the balloon catheter system 10 together with the aforementioned balloon catheter 15, to be specific, the heating device 70, the supply device 74, and the agitation device 75, are described.

The illustrated heating device 70 is electrically connected to the electrode for high-frequency current conduction 41 via a connection cable 56a and the lead wire for high-frequency current conduction 42. High-frequency power generated by the heating device 70 is supplied to the electrode for high-frequency current conduction 41 through the connection cable 56a and the lead wire for high-frequency current conduction 42. The heating device 70 has a high-frequency current conduction controller 71 that controls high-frequency current conduction to the electrode for high-frequency current conduction 41. In the illustrated example, the high-frequency current conduction controller 71 controls the high-frequency current conduction to the electrode for high-frequency current conduction 41 to adjust an output from the heating member 40. In addition, the high-frequency current conduction controller 71 is electrically connected to the connection cable 56a and the lead wire for temperature sensor 47, so that the high-frequency current conduction to the electrode for high-frequency current conduction 41 can be controlled based on information on a temperature of the liquid in the balloon 25 acquired by the temperature sensor 45. The high-frequency current from the electrode for high-frequency current conduction 41 returns to the heating device 70 through a human body (patient) and the counter electrode 77 attached to the back of the human body (patient). Since the high-frequency current generated by the heating device 70 is an alternating current, a flow direction changes at a certain frequency (e.g., 1.8 MHz).

The heating device 70 is constituted by a hardware such as a CPU, for example. One or more of the high-frequency current conduction controller 71 and other components included in the heating device 70 may be constituted by a separate hardware. At least a part of the heating device 70 may be constituted by a software. A part of the heating device 70 may be physically separated from the other part of the heating device 70. A component of the heating device 70 may be able to cooperate with another component thereof by communication through a network. A component of the heating device 70 may be on a device such as a server or database in the cloud, which can communicate with another component of the heating device 70 through an external network.

Next, the supply device 74 is described. The supply device 74 supplies a liquid into the liquid delivery path LP. The supply device 74 can inflate the balloon 25, as shown in Fig. 2, by supplying a liquid to the balloon 25 through the liquid delivery path LP. Also, the supply device 74 can deflate the balloon 25 by discharging the liquid from the balloon 25 through the liquid delivery path LP. A syringe can be used as the supply device 74 as illustrated. A pump or the like can also be used as the supply device 74. The liquid to be supplied to the liquid delivery path LP is preferably a contrast agent or a contrast agent diluted with saline in order that the balloon 25 inflated with the liquid can be seen in a radiographic image.

Next, the agitation device 75 is described. The agitation device 75 is provided for vibrating to agitate a liquid in the balloon 25. By agitating the liquid in the balloon 25, the liquid heated by the heating member 40 can be diffused to equalize a temperature of the liquid in the balloon 25. As a result, a surface temperature of the balloon 25 can be controlled. The agitation device 75 repeats supply of liquid to the liquid delivery path LP and discharge of liquid from the liquid delivery path LP. Thus, supply of liquid from the liquid delivery path LP into the balloon 25 and discharge of liquid from inside the balloon 25 to the liquid delivery path LP are repeated, so that the liquid in the balloon 25 is vibrated and agitated. A pump selected from the group consisting of a roller pump, a diaphragm pump, a bellows pump, a vane pump, a centrifugal pump, and a pump comprising a piston and a cylinder in combination can be used as the agitation device 75.

The amount of liquid to be supplied to the liquid delivery path LP and the amount of liquid to be discharged from the liquid delivery path LP may be a predetermined amount (e.g., between 0.5 ml or more and 1.5 ml or less). Supply of liquid to the liquid delivery path LP and discharge of liquid from the liquid delivery path LP may be repeated according to a regular cycle (e.g., between one and three times per second). The amount of liquid to be supplied to the liquid delivery path LP and the amount of liquid to be discharged from the liquid delivery path LP may be adjusted by a control signal from an agitation control device 76 or by a direct input from an operator. Similarly, a cycle at which a liquid is supplied to the liquid delivery path LP and a liquid is discharged from the liquid delivery path LP may be adjusted by a control signal from the agitation control device 76 or by a direct input from an operator.

In order to control a surface temperature of the balloon 25 to an ideal temperature in a reliable manner, it is necessary to efficiently diffuse a liquid heated by the heating member 40 in the balloon 25 in a reliable manner. In order to diffuse the heated liquid efficiently in a reliable manner, most (or all) of the liquid flowing from the liquid delivery path LP into the balloon 25 is desired to be directed to the heating member 40 in a reliable manner, during the supply of liquid by the agitation device 75 from the liquid delivery path LP into the balloon 25. However, if a pressing force is applied to the balloon, for example, so that the inside shaft 35 is curved during an operation, there is a possibility that a direction along which the distal end 30a of the outside shaft 30 and the heating member 40 are aligned is significantly displaced from an ejection direction along which the liquid is ejected from the distal end 30a of the outside shaft 30 (a direction along the center axis 30X of the outside shaft 30 at the distal end 30a). When the liquid is supplied into the balloon 25 at this angle, most or all of the liquid flowing from the liquid delivery path LP into the balloon 25 is not directed to the heating member 40, whereby most or all of the heated liquid in the vicinity of the heating member 40 cannot be diffused. As a result, a surface temperature of the balloon 25 cannot be raised as desired. In addition, since a temperature of the liquid in the vicinity of the temperature sensor 45 is maintained to be high, electric energy is not sufficiently supplied from the heating device 70 to the heating member 40 although a surface temperature of the balloon 25 does not reach a desired temperature. Also, in this case, there is a possibility that a surface temperature of the balloon 25 cannot be raised. Namely, unless the liquid heated by the heating member 40 can be efficiently diffused in a reliable manner, it is difficult to adjust a surface temperature of the balloon 25 to an ideal temperature in a reliable manner.

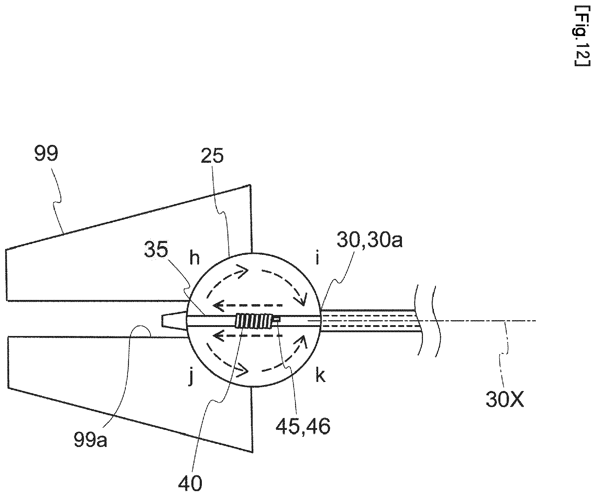

Fig. 12 shows the balloon catheter 15, with the balloon 25 being perpendicularly pressed against an opening of an ostia venarum pulmonalium of a pseudo living-body 99. In the example shown in Fig. 12, a direction along which the force is applied to the balloon 25 is a direction along the center axis 30X at the distal end 30a of the outside shaft 30. The inside shaft 35 is not curved between the distal end 30a of the outside shaft 30 and the heating member 40. Thus, the distal end 30a of the outside shaft 30 and the heating member 40 are aligned along the ejection direction (the direction along the center axis 30X of the outside shaft 30 at the distal end 30a) along which the liquid is ejected from the distal end 30a of the outside shaft 30.

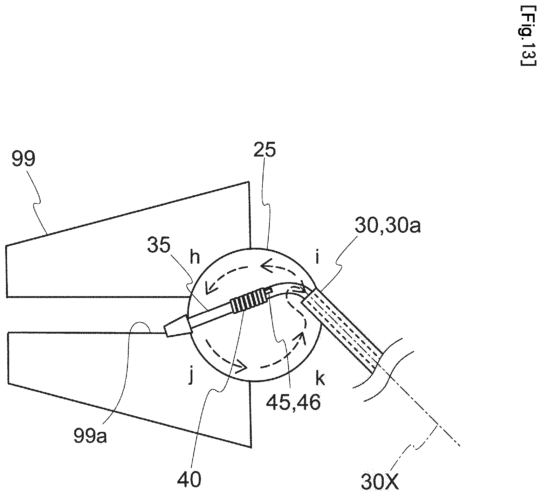

Fig. 13 shows the balloon catheter 15, with the balloon 25 being pressed against an opening of an ostia venarum pulmonalium of the pseudo living-body 99 at an angle between 45 degrees and 60 degrees. In the example shown in Fig. 13, a direction along which the force is applied to the balloon 25 intersects the direction along the center axis 30X at the distal end 30a of the outside shaft 30. The inside shaft 35 is largely curved between the distal end 30a of the outside shaft 30 and the heating member 40. Thus, the distal end 30a of the outside shaft 30 and the heating member 40 are not aligned along the ejection direction, along which the liquid is ejected from the distal end 30a of the outside shaft 30.

In Figs. 12 and 13, a flow of the liquid (agitation flow) ejected from the distal end 30a of the outside shaft 30 into the balloon 25 is indicated by arrows.

As can be understood from Fig. 12, when the inside shaft 35 is not curved between the distal end 30a of the outside shaft 30 and the heating member 40, much of the liquid ejected from the liquid delivery path LP into the balloon 25 surrounds the inside shaft 35 moving toward the heating member 40 and diffusing much of the heated liquid in the vicinity of the heating member 40 to surround the heating member 40 anew. The diffused liquid moves toward the surface of the balloon 25 to heat the surface of the balloon 25. The liquid which has surrounded the heating member 40 anew is heated by the heating member 40. In the example shown in Fig. 12, the liquid heated by the heating member 40 is evenly diffused in the balloon 25, whereby the inside of the balloon 25 and the surface of the balloon 25, i.e., areas indicated by h, i, j, k in Fig. 12 have substantially the same temperature as a whole. In addition, since the liquid heated by the heating member 40 is efficiently diffused, it is easy to adjust a surface temperature of the balloon 25 to a target temperature by controlling supply of electric energy to the heating member 40. This can significantly improve an ablation therapy effectiveness.

On the other hand, as can be understood from Fig. 13, when the inside shaft 35 is largely curved between the distal end 30a of the outside shaft 30 and the heating member 40, the liquid ejected from the liquid delivery path LP into the balloon 25 is deflected from the heating member 40 and moves toward the surface of the balloon 25 without being heated by the heating member 40. In the example shown in Fig. 13, the liquid heated by the heating member 40 cannot be efficiently diffused. Specifically, most (or all) of the liquid heated by the heating member 40 cannot be diffused by the agitation flow. Alternatively, the heated liquid cannot be evenly diffused in the balloon 25. Thus, conduction of heat generated by the heating member 40 relies on heat conduction in the balloon 25, which makes the temperature of the entire balloon 25 low and unstable. Alternatively, the surface temperature of the balloon 25 becomes uneven. For example, when the liquid flows in the balloon 25 as shown in Fig. 13, temperatures of surface areas indicated by i and h may be lower than those of areas indicated by j and k. This makes it difficult to control a surface temperature of the balloon 25 to a target temperature. As a result, an ablation therapy cannot be performed as desired.

In consideration of these issues, the balloon catheter 15 in this embodiment is designed to efficiently diffuse the liquid heated by the heating member 40 in a reliable manner. Specifically, the outside shaft 30 extends into the balloon 25. Since the outside shaft 30 restrains the part of the inside shaft 35 extending inside the outside shaft 30 from being bent by the outside shaft 30, it is possible to restrain the inside shaft 35 from being curved between the distal end 30a of the outside shaft 30 and the heating member 40 with the outside shaft 30 extending into the balloon 25 so that the distal end 30a of the outside shaft 30 and the heating member 40 are close to each other. Thus, even when a force is applied to the balloon 25 or the inside shaft 35 in a direction intersecting the direction along the center axis 30X at the distal end 30a of the outside shaft 30, much of the liquid supplied from the liquid delivery path LP into the balloon 25 can be directed to the heating member 40 in a reliable manner. In addition, the heated liquid in the vicinity of the heating member 40 can be efficiently diffused in a reliable manner. Moreover, the liquid supplied from the liquid delivery path LP into the balloon 25 can be efficiently heated by the heating member 40 in a reliable manner.

Further, since the outside shaft 30 extends into the balloon 25 so that the distal end 30a of the outside shaft 30 and the heating member 40 are close to each other, much more of the liquid supplied from the liquid delivery path LP into the balloon 25 can be directed to the heating member 40. Namely, not only when a force is applied to the balloon 25 or the inside shaft 35 in a direction intersecting the direction along the center axis 30X at the distal end 30a of the outside shaft 30, but also when a force is applied to them in the aforementioned direction along the center axis 30X, most (or all) of the liquid supplied from the liquid delivery path LP into the balloon 25 can be directed to the heating member 40. Thus, diffusion efficiency of the heated liquid in the vicinity of the heating member 40 can be improved, and heating efficiency of the liquid supplied from the liquid delivery path LP into the balloon 25 can also be improved.

In the illustrated example, a distance between the distal end 30a of the outside shaft 30 and the proximal end 40b of the heating member 40 when the balloon 25 is loosened (and when the balloon 25 is inflated), is between 0 mm and 5 mm. Since the distal end 30a of the outside shaft 30, which is a supply outlet of liquid, and the proximal end 40b of the heating member 40 are so close to each other that the inside shaft 35 can be effectively restrained from being curved between the distal end 30a of the outside shaft 30 and the heating member 40.

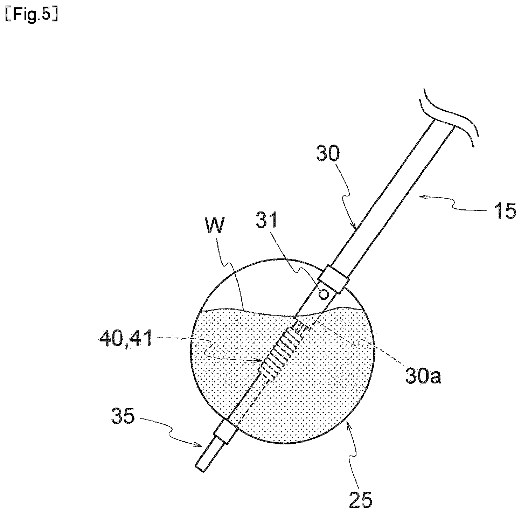

As shown in Figs. 1 to 4, the part of the outside shaft 30 extending inside of the balloon 25 is provided with a through hole 31 which passes through the outside shaft 30 in a direction along a radial direction of a circle centered on the center axis 30X. When inflating the balloon 25 for balloon preparation, it is necessary to discharge a gas (to vent air) from the balloon 25 during the supply of the liquid into the balloon 25. Typically, air venting is performed by sucking the gas in the balloon 25 at the distal end 30a of the outside shaft 30 into the liquid delivery path LP. However, since the outside shaft 30 extends into the balloon 25, as shown in Fig. 5, an increase in the amount of liquid in the balloon 25 causes the distal end 30a of the outside shaft 30 to be immersed in the liquid W in the balloon 25. In this case, it is impossible to discharge the gas in the balloon 25 through the distal end 30a. Thus, the through hole 31 for venting air is provided between the distal end 30a of the outside shaft 30 and a connection part of the outside shaft 30 to which the proximal end 25b of the balloon 25 is connected. Thus, even when the distal end 30a of the outside shaft 30 is immersed in the liquid W in the balloon 25, air can be vented through the through hole 31.

The illustrated example is also designed to ensure that electrical energy is properly supplied to the heating member 40. Specifically, as shown in Fig. 2, the thermosensitive part 46 of the temperature sensor 45 is disposed close to the distal end 30a of the outside shaft 30. To be specific, the thermosensitive part 46 is located at a position closer to the distant end than the distal end 30a of the outside shaft 30 by 0 mm to 5 mm. In the illustrated example, the thermosensitive part 46 is disposed at the position where the proximal end 40b of the heating member 40 is located. This position is where the liquid flowing from the liquid delivery path LP into the balloon 25 passes, before the liquid surrounds the heating member 40 (thus before the liquid is heated by the heating member 40). Thus, the temperature sensor 45 can acquire information on a temperature of the liquid which comes from the liquid delivery path LP toward the heating member 40 (i.e., a temperature of the liquid which is going to be heated by the heating member 40). By supplying electric energy to the heating member 40 based on such information, the liquid which is going to surround the heating member 40 anew can be heated to a proper temperature. As a result, a surface temperature of the balloon 25 can be suitably controlled.

Next, an example of the use of the balloon catheter system 10 as structured above is described.

The inside shaft 35 is first relatively moved with respect to the outside shaft 30 to the distal side (distant side) in the longitudinal direction LD, so that the balloon 25 is stretched as shown in Fig. 3. The outside shaft 30 and the inside shaft 35 can be relatively moved by operating the first handle part 51 and the second handle part 52 of the handle 50. The catheter body 20 with the balloon 25 stretched is inserted into the patient's body. When the catheter body 20 is inserted into the patient's body, the balloon 25 is not filled with a liquid.

Next, the distal end of the catheter body 20 is guided close to a target site (diseased area), and then the inside shaft 35 is relatively moved with respect to the outside shaft 30 to the proximal side (near side) in the longitudinal direction LD, so that the balloon 25 is loosened. Then, the valve 58 is operated so as to communicate the supply device 74 with the liquid delivery path LP of the catheter body 20 through the handle 50. Thereafter, the supply device 74 is operated to let a liquid to flow into the liquid delivery path LP, so that the balloon 25 is inflated with the liquid as shown in Fig. 2. At this time, the air inside the balloon 25 is ventilated through the liquid delivery path LP, so that the balloon 25 is filled with the liquid.

Then, the valve 58 is operated to shut off the supply device 74 from the liquid delivery path LP, and to communicate the agitation device 75 with the liquid delivery path LP. The agitation device 75 is controlled by a control signal from the agitation control device 76, in such a manner that the agitation device 75 repeats supply of a predetermined amount of liquid to the liquid delivery path LP and discharge of a predetermined amount of liquid from the liquid delivery path LP according to a regular cycle. This results in repeated injection of the predetermined amount of liquid from the liquid delivery path LP into the balloon 25 and suction of the predetermined amount of liquid from inside the balloon 25 to the liquid delivery path LP according to a regular cycle. Thus, the liquid in the balloon 25 is vibrated and agitated.

Also, a liquid temperature in the balloon 25 is stabilized by controlling the heating member 40 by means of the high-frequency current conduction controller 71 of the heating device 70. Specifically, by means of the heating device 70, high-frequency voltage is applied between the electrode for high-frequency current conduction 41 forming the heating member 40 and the counter electrode 77 disposed outside the body of a patient. As a result, a high-frequency current flows between the coil electrode 41 and the counter electrode 77. Since the size of the electrode for high-frequency current conduction 41 is significantly smaller than that of the counter electrode 77, a current density around the electrode for high-frequency current conduction 41 is high, and the liquid around the electrode for high-frequency current conduction 41 is heated by Joule heating.

The liquid in the balloon 25 is agitated while being heated as described above. Then, the balloon 25 containing the heated liquid is pressed against the target site to ablate it.

Most (or all) of the liquid ejected from the distal end 30a of the outside shaft 30 is directed to the heating member 40 located close to the distal end 30a. Then, the liquid diffuses most (or all) of the heated liquid in the vicinity of the heating member 40 and surrounds the heating member 40 anew to be heated by the heating member 40. In this manner, the liquid flowing from the liquid delivery path LP into the balloon 25 efficiently diffuses the liquid heated by the heating member 40, and is also efficiently heated by the heating member 40.

The temperature sensor 45 disposed close to the distal end 30a of the outside shaft 30 acquires information on a temperature of the liquid which comes from the liquid delivery path LP toward the heating member 40 (i.e., a temperature of the liquid which is going to be heated by the heating member 40). High-frequency current conduction by the heating device 70 to the electrode for high-frequency current conduction 41 is controlled based on the information. As a result, the liquid surrounding the heating member 40 anew is heated to a proper temperature and a surface temperature of the balloon 25 is properly adjusted.

By using the balloon catheter system 10 described above, it is possible for an operator to proceed the procedure while adjusting a surface temperature of the balloon, which is one of the most important factors in the ablation therapy, to an ideal temperature. This can significantly improve the ablation therapy effect.

Upon completion of the ablation to the target site, energy supply to the heating member 40 is stopped. In addition, the valve 58 is operated so that the supply device 74 is communicated with the liquid delivery path LP in the catheter body 20 through the handle 50, and that the agitation device 75 is shut off from the liquid delivery path LP. Then, the liquid is discharged by means of the supply device 74 from the liquid delivery path LP to deflate the balloon 25. Then, the second handle part 52 is operated to stretch the deflated balloon 25 as shown in Fig. 3. After that, the catheter body 20 with the stretched balloon 25 is pulled out of the patient's body. In this manner, the procedure using the balloon catheter system 10 is completed.

### <Second Embodiment>

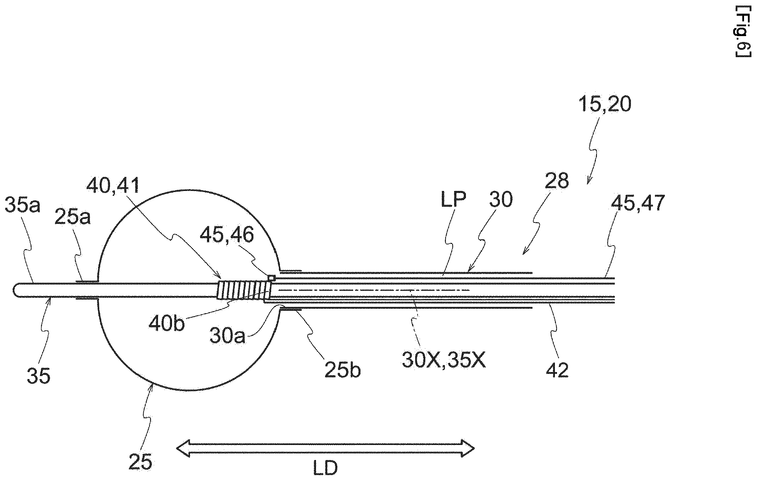

Next, a balloon catheter 15 according to a second embodiment is described with reference to Fig. 6. Fig. 6 is a view showing a distal end portion of the balloon catheter 15 according to the second embodiment, with the balloon 25 and the outside shaft 30 being cut along the longitudinal direction LD.

The example shown in Fig. 6 differs from the balloon catheter 15 shown in Figs. 1 to 5 in that the outside shaft 30 does not extend into the balloon 25, and that the outside shaft 30 is not provided with the through hole 31 for venting air. In addition, it differs from the balloon catheter 15 shown in Figs. 1 to 5 in that the heating member 40 is not located at the midpoint of the balloon 25 in the direction of the center axis 35X of the inside shaft 35. The other configurations are substantially the same as those of the balloon catheter 15 shown in Figs. 1 to 5. In the example shown in Fig. 6, the same numerals are given to the same parts as those of the first embodiment shown in Figs. 1 to 5, and detailed description is omitted.

As described above, in the example shown in Fig. 6, the outside shaft 30 does not extend into the balloon 25 and its distal end 30a is not inside the balloon 25. However, the heating member 40 is located closer to the proximal end 25b of the balloon 25, as compared with the case shown in Figs. 1 to 5. Specifically, when the balloon 25 is loosened (and when the balloon 25 is inflated), the midpoint of the heating member 40 in the direction of the center axis 35X of the inside shaft 35 is positioned closer to the proximal end 25b than the midpoint between the proximal end 25b of the balloon 25 and the distal end 25a thereof. A distance between the distal end 30a of the outside shaft 30 and the proximal end 40b of the heating member 40 is between 0 mm and 5 mm.

Also, in such a balloon catheter 15, the inside shaft 35 is restrained from being curved between the distal end 30 of the outside shaft 30 and the heating member 40. Thus, even when a force is applied to the balloon 25 or the inside shaft 35 in a direction intersecting the direction along the center axis 30X at the distal end 30a of the outside shaft 30, most (or all) of the liquid supplied from the liquid delivery path LP into the balloon 25 can be directed to the heating member 40 in a reliable manner. In addition, the heated liquid in the vicinity of the heating member 40 can be efficiently diffused in a reliable manner. Moreover, the liquid supplied from the liquid delivery path LP into the balloon 25 can be efficiently heated by the heating member 40 in a reliable manner.

### <Third Embodiment>

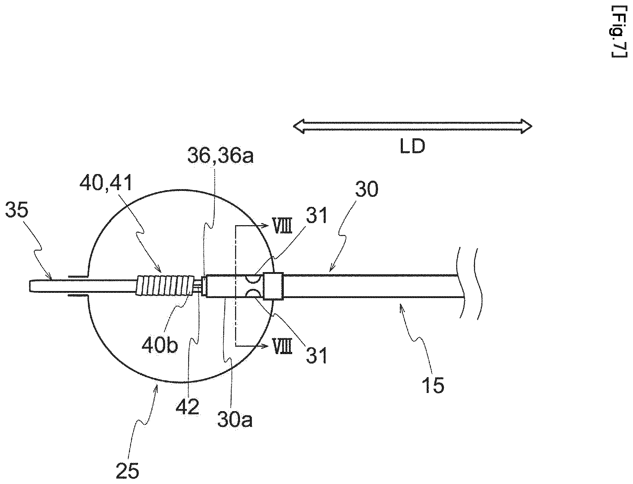

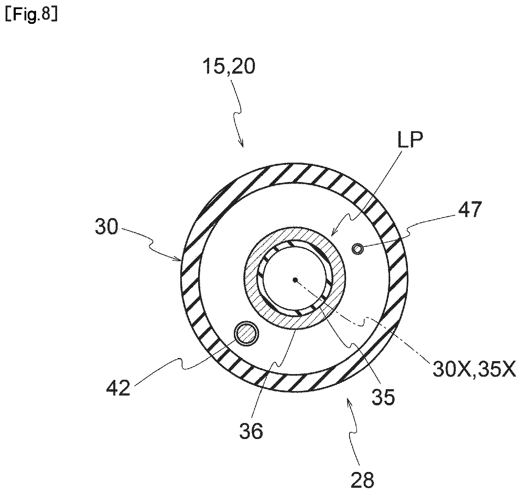

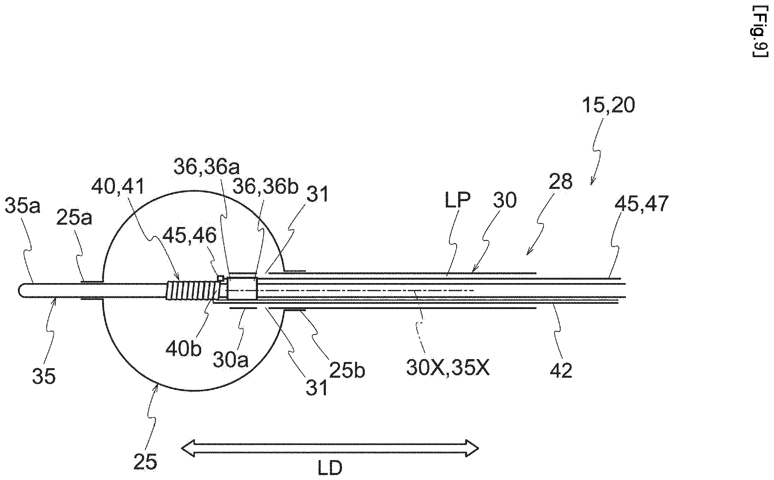

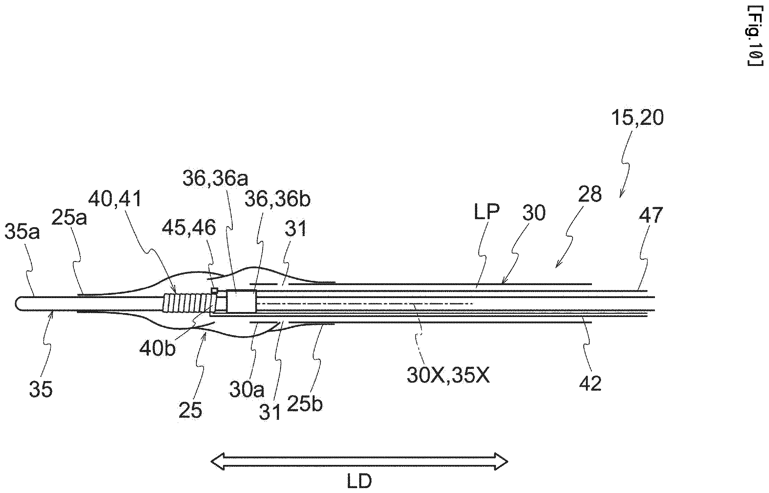

Next, a balloon catheter 15 according to a third embodiment is described with reference to Figs. 7 to 10. Fig. 7 is a view showing a distal end of the balloon catheter 15 according to the third embodiment. Fig. 8 is a cross-sectional view of the balloon catheter 15 shown in Fig. 7 along a VIII-VIII line. Fig. 9 is a view corresponding to Fig. 2, showing a distal end portion of the balloon catheter 15 shown in Fig. 7, with the balloon 25 and the outside shaft 30 being cut along the longitudinal direction LD. Fig. 10 is a view corresponding to Fig. 3, showing the distal end portion 15 of the balloon catheter shown in Fig. 9, with the balloon being deflated and stretched.

The example shown in Figs. 7 to 10 differs from the balloon catheter 15 shown in Figs. 1 to 5 in that the inside shaft 35 is provided with a reinforcement tube 36. The other configurations are substantially the same as those of the balloon catheter shown in Figs. 1 to 5. In the example shown in Figs. 7 to 10, the same numerals are given to the same parts as those of the first embodiment shown in Figs. 1 to 5, and detailed description thereof is omitted.

The third balloon catheter 15 in the third embodiment is designed to more effectively restrain the inside shaft 35 from being curved between the distal end 30a of the outside shaft 30 and the heating member 40. Specifically, as shown in Figs. 7 and 8, the balloon catheter 15 has the reinforcement tube 36 covering an outer circumferential surface of the inside shaft 35 in the balloon 25. As can be understood from Fig. 9, the reinforcement tube 36 extends from between the proximal end 40b of the heating member 40 and the distal end 30a of the outside shaft 30 to the proximal side. As shown in Fig. 9, the reinforcement tube 36 extends into the outside shaft 30 with the balloon 25 being loosened (or with the balloon being inflated). Such a reinforcement tube 36 can further effectively restrain the inside shaft 35 from being curved between the distal end 30a of the outside shaft 30 and the heating member 40. As a result, even when a force is applied to the balloon 25 or the inside shaft 35 in a direction intersecting the direction along the center axis 30X at the distal end 30a of the outside shaft 30, most (or all) of the liquid supplied from the liquid delivery path LP into the balloon 25 can be directed to the heating member 40 in a reliable manner. In addition, the heated liquid in the vicinity of the heating member 40 can be efficiently diffused in a reliable manner. Moreover, the liquid supplied from the liquid delivery path LP into the balloon 25 can be efficiently heated by the heating member 40 in a reliable manner.

As described above, the outside shaft 30 restrains a part of the inside shaft 35 extending in the outside shaft 30 from being curved with respect to the center axis 30X of the outside shaft 30. Thus, since the reinforcement tube 36 extends into the outside shaft 30, the inside shaft 35 can be restrained from being curved with respect to the aforementioned center axis 30X, continuously from the inside of the outside shaft 30 to a distal end 36a of the reinforcement tube 36.

A distance between the proximal end 40b of the heating member 40 and the distal end 36a of the reinforcement tube 36 is preferably between 0 mm and 5 mm. Such positioning of the reinforcement tube 36 allows the reinforcement tube 36 to effectively restrain the inside shaft 35 from being curved.

The reinforcement tube 36 can be fixed to the inside shaft 35 in a manner using welding or adhesion using tape or glue.

The reinforcement tube 36 can be made of polymers such as polyurethane, polyamide, polyimide, and fluoropolymers, etc.. However, the material of the reinforcement tube 36 is not specifically limited, as long as it has certain rigidity and flexibility.

A thickness of the reinforcement tube 36 is suitably determined in consideration of rigidity and flexibility required for the distal end portion of the catheter body 20. The reinforcement tube 36 preferably has a large thickness to have rigidity to the extent that it has plasticity. On the other hand, when the thickness of the reinforcement tube 36 is excessively large, a volume of the lumen between the outside shaft 30 and the inside shaft 35 decreases, which makes it difficult to transmit vibrations of the liquid filled in the lumen to the liquid in the balloon 25. Thus, the thickness of the reinforcement tube 36 is preferably between 0.5 mm and 2.0 mm, for example, although it depends on a difference between the internal diameter of the outside shaft 30 and the external diameter of the inside shaft 35.

As shown in Fig. 10, a length of the reinforcement tube 36 is determined in such a manner that a proximal end 36b of the reinforcement tube 36 is still inside the outside shaft 30, even when the inside shaft 35 is relatively moved as much as possible with respect to the outside shaft 30 to the distal side in the longitudinal direction LD so as to stretch the balloon 25. This specific example can prevent the reinforcement tube 36 from moving entirely outside of the outside shaft 30, when the inside shaft 35 is relatively moved with respect to the outside shaft 30 to stretch the balloon 25. This prevents the proximal end 36b of the reinforcement tube 36 from being positioned outside the outside shaft 30. Therefore, when the inside shaft 35 is relatively moved with respect to the outside shaft 30 to loosen the balloon 25, the proximal end 36b of the reinforcement tube 36 and the distal end 30a of the outside shaft 30 are prevented from interfering with each other to disturb the relative movement between the inside shaft 35 and the outside shaft 30.

On the other hand, when the reinforcement tube 36 is excessively long, the lumen between the outside shaft 30 and the inside shaft 35 narrowed by the reinforcement tube 36 will be very long, whereby vibrations of the liquid in the balloon 25 tend to be attenuated. As a result, there is a possibility that the heated liquid in the vicinity of the heating member 40 cannot be effectively diffused so that a surface temperature of the balloon 25 cannot be raised as desired. There is also a possibility that rigidity of the distal end portion of the catheter body 20 increases too much, which makes it difficult to move the distal end portion of the catheter body 20 in the curved sheath. Considering the above, the length of the reinforcement tube 36 is preferably between 5 mm and 20 mm, for example.

Some specific examples included in the aforementioned embodiment are described hereunder, in comparison with a comparative example. In the description of the below specific examples and the drawings used for describing the below specific examples and the comparative example, there are parts similarly structured to those described in the above description, and the former are designated by the same numerals as the latter and will not be described in detail repeatedly.

### <Specific Example 1>

In order to produce the balloon catheter 15 of Specific Example 1, the polyurethane balloon 25 having a diameter of 30 mm and a thickness of 20 µm was produced by blow molding.

A polyurethane tube having an external diameter 3.6 mm, an internal diameter of 3.0 mm and a total length of 1000 mm was used as the outside shaft 30. The through hole 31 for venting air was made in the outside shaft 30 at a position close to its distal end 30a. A polyamide tube having an external diameter of 1.6 mm, an internal diameter of 1.2 mm and a total length of 1100 mm was used as the inside shaft 35.

The coiled electrode for high-frequency current conduction 41 having a length of 13 mm was formed by partially peeling the electric insulation protective covers provided on the lead wire for high-frequency current conduction 42 and the lead wire for temperature sensor 47, and winding the lead wire for high-frequency current conduction 42 around the inside shaft 35 with the lead wire for temperature sensor 47 being sandwiched between the lead wire for high-frequency current conduction 42 and the inside shaft 35. At the proximal end 40b of the electrode for high-frequency current conduction 41, the lead wire for high-frequency current conduction 42 and lead wire for temperature sensor 47 were fixed to each other by welding in order to form a thermocouple as the temperature sensor 45.

The inside shaft 35 was inserted into the lumen of the outside shaft 30. The inside shaft 35 was slidable in the lumen relative to the outside shaft 30. The distal end 25a of the balloon 25 was fixed on the distal end 35a of the inside shaft 35. The distal end 30a of the outside shaft 30 was inserted into the balloon 25 from the proximal end 25b of the balloon 25. The proximal end 25b of the balloon 25 was fixed closer to the proximal end of the outside shaft 30 than the distal end 30a of the outside shaft 30 (more specifically, than the through hole 31).

The polycarbonate handle 50 was provided on the rear ends of the outside shaft 30 and the inside shaft 35. The handle 50 was composed of the first handle part (front handle part) 51 connected to the outside shaft 30, and the second handle part (rear handle part) 52 connected to the inside shaft 35. The inside shaft 35 could slide in the outside shaft 30 by sliding the second handle 52 with respect to the first handle 51 so as to deform the balloon 25.

A position of the heating member 40 on the inside shaft 35 was determined in such a manner that the midpoint of the heating member 40 in the direction of the center axis 35X of the inside shaft 35 was positioned at the midpoint between the proximal end 25b of the balloon 25 and the distal end 25a thereof, when the balloon 25 was loosened. A position of the proximal end 25b of the balloon 25 on the outside shaft 30 was determined in such a manner that a distance between the proximal end 40b of the heating member 40 and the distal end 30a of the outside shaft 30 was 5 mm.

### <Specific Example 2>

The balloon catheter 15 was produced similarly to the one according to Specific Example 1, except that the proximal end 25b of the balloon 25 was fixed on the distal end 30a of the outside shaft 30 and the outside shaft 30 did not extend into the balloon 25, that the heating member 40 was disposed closer to the proximal end 25b of the balloon 25 compared to Specific Example 1, and that the through hole 31 for venting air was not made in the outside shaft 30. In the balloon catheter 15 in Specific Example 2, a position of the heating member 40 on the inside shaft 35 was determined in such a manner that a distance between the proximal end 40b of the heating member 40 and the distal end 30a of the outside shaft 30 was 5 mm, when the balloon 25 was loosened.

### <Specific Example 3>

The balloon catheter 15 was produced similarly to the one according to Specific Example 1, expect that the reinforcement tube 36 was provided on the inside shaft 35.

Specifically, the polyurethane reinforcement tube 36 having a length of 20 mm, an external diameter of 2.4 mm and an internal diameter of 1.6 mm was fixed by welding on the outer circumferential surface of the inside shaft 35 on the proximal side of the heating member 40. A position of the reinforcement tube 36 on the inside shaft 35 was determined in such a manner that the reinforcement tube 36 protruded from the distal end 30a of the outside shaft 30 by 5 mm and was received in the outside shaft 30 by 15 mm, when the balloon 25 was loosened.

### <Comparative Example>

The balloon catheter 15 was produced similarly to the one according to Specific Example 1, except that the proximal end 25b of the balloon 25 was fixed on the distal end 30a of the outside shaft 30 and the outside shaft 30 did not extend into the balloon 25, and that the through hole 31 for venting air was not made in the outside shaft 30. In the balloon catheter 15 in Comparative Example, a distance between the proximal end 40b of the heating member 40 and the distal end 30a of the outside shaft 30 was 8.5 mm, when the balloon 25 was loosened.

### <Comparative Experiment of Balloon Surface Temperature>

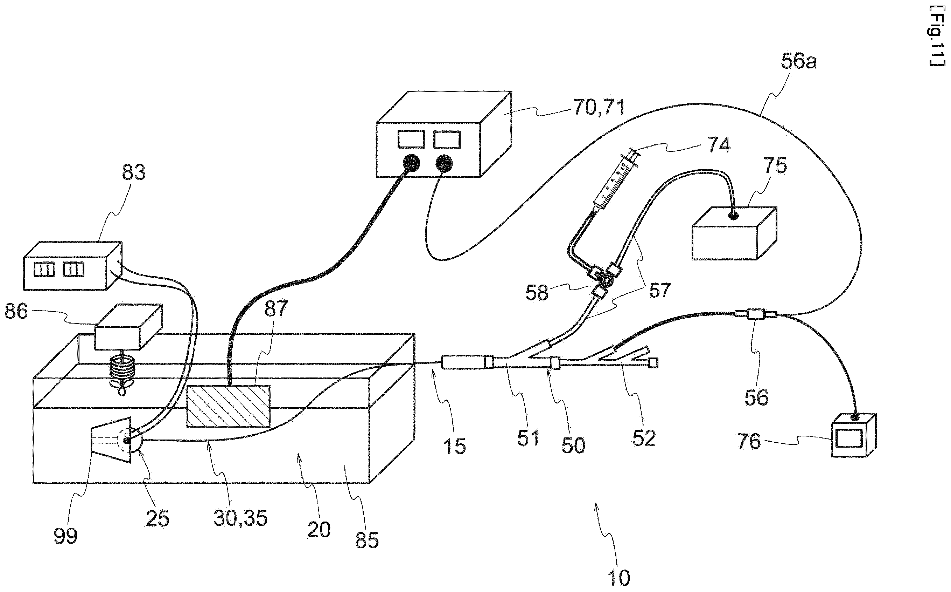

The balloon catheters 15 produced in these manners were used to apply ablation therapy to a pseudo living-body 99 that representing a left atrium ostia venarum pulmonalium of a human body (see Fig. 11). The pseudo living-body 99, which was produced by molding an acryl-based resin, was immersed in a saline solution in a water tank 85. During the experiment, the saline solution in the water tank 85 was agitated using a pipe heater 86 with an agitator, and a temperature of the saline solution was maintained at 36.5°C. The counter electrode 87 for generating a high-frequency current between the counter electrode 87 and the electrode for high-frequency current conduction 41 of the catheter body 20 was located on a sidewall of the water tank 85. The saline solution in the water tank 85 was a solution of 0.9 wt% of salt (sodium chloride) dissolved in water.

A liquid supplied from the supply device 74 to the liquid delivery path LP and the balloon 25 was a mixture of a saline solution of 0.9 wt% of salt (sodium chloride) dissolved in water and a contrast agent for X-ray contrast. An injection amount of liquid into the balloon 25 had two levels, i.e., 10 mL and 20 mL, which are generally used in actual ablation therapy. The contrast mixed in the liquid was Omnipark (registered trademark) manufactured by Daiichi Sankyo Company, Ltd. A dilution ratio of the contrast (contrast dilution ratio) had two levels, i.e., 1:2 and 1:3. The contrast dilution ratio here means the ratio of "saline solution volume : contrast agent volume".

A surface temperature of the balloon 25, when the balloon 25 was pressed against an opening of the ostia venarum pulmonalium perpendicularly with respect to the opening of the ostia venarum pulmonalium (namely, when an orientation of the force applied from the pseudo living-body 99 to the balloon 25 was along the center axis 30X of the outside shaft 30 at the distal end 30a of the outside shaft 30 (which is referred to also as "first case" hereunder)), was measured. In addition, a surface temperature of the balloon 25, when the balloon 25 was pressed against the opening of the ostia venarum pulmonalium of the pseudo living-body 99 at an angle between 45 degrees and 60 degrees with respect to the opening of the ostia venarum pulmonalium (namely, when an orientation of the force applied from the pseudo living-body 99 to the balloon 25 was at an angle between 30 degrees and 45 degrees with respect to the direction along the center axis 30X of the outside shaft 30 at the distal end 30a of the outside shaft 30 (which is referred to also as "second case")), was measured.

### <Comparative Experiment Results>

### <<Comparative Experiment Result in First Case>>

In the catheter 15 according to Comparative Example, in the first case, an alignment direction of the distal end 30a of the outside shaft 30 and the heating member 40 was along the center axis 30X of the outside shaft 30 at the distal end 30a of the outside shaft 30. In this case, by adjusting a temperature of the heating member 40 to 70°C, the balloon 25 could have a surface temperature of 65°C.

### <<Comparative Experiment Result in Second Case>>

On the other hand, in the second case, the inside shaft 35 of the catheter 15 according to Comparative Example was curved between the distal end 30a of the outside shaft 30 and the heating member 40, and an alignment direction of the distal end 30a of the outside shaft 30 and the heating member 40 was largely deflected from the center axis 30X of the outside shaft 30 at the distal end 30a of the outside shaft 30. In this case, the balloon 25 had a surface temperature of 60°C when adjusting a temperature of the heating member 40 to 70°C. Namely, in the second case, although the temperature of the heating member 40 was adjusted to the same temperature as that of the heating member 40 in the first case, the surface temperature of the balloon 25 was lower by 5°C compared to the above first case. The reason is considered to be as follows. Since the alignment direction of the distal end 30a of the outside shaft 30 and the heating member 40 was largely deflected from the center axis 30X of the outside shaft 30 at the distal end 30a of the outside shaft 30, most of the liquid supplied from the liquid delivery path LP was not directed to the heating member 40, but flowed toward the balloon 25 without diffusing the heated liquid in the vicinity of the heating member 40 or being heated by the heating member 40.

### <<Result of Specific Example 1 in First Case>>

In the balloon catheter 15 according to Specific Example 1, in the first case, an alignment direction of the distal end 30a of the outside shaft 30 and the heating member 40 was along the center axis 30X of the outside shaft 30 at the distal end 30a of the outside shaft 30. In this case, by adjusting a temperature of the heating member 40 to 65°C, the balloon 25 had a surface temperature of 65°C. Namely, the balloon 25 could have a surface temperature of 65°C by adjusting the temperature of the heating member 40 to a temperature lower than that of the heating member 40 in Comparative Example. The reason is considered to be as follows. Since the distal end 30a of the outside shaft 30 in Specific Example 1 was closer to the heating member 40 compared to Comparative Example, a larger part of the liquid supplied from the liquid delivery path LP was directed to the heating member 40, and could efficiently diffuse the heated liquid in the vicinity of the heating member 40, compared to the first case of Comparative Example. In addition, since a larger part of the liquid supplied from the liquid delivery path LP was directed to the heating member 40 compared to the first case of Comparative Example, the liquid was efficiently heated by the heating member 40.

### <<Result of Specific Example 1 in Second Case>>

In the second case, although the inside shaft 35 of the catheter 15 according to Specific Example 1 was curved between the distal end 30a of the outside shaft 30 and the heating member 40, such curve was gentle compared to the inside shaft 35 in Comparative Example in the second case. The balloon 25 had a surface temperature of 64°C when adjusting the temperature of the heating member 40 to 65°C. Namely, the surface temperature of the balloon 25 was lower by 1°C only, compared to the first case of Specific Example 1. The reason of a smaller difference between the surface temperatures of the balloon 25 in the first and second cases of Specific Example 1 compared to the first and second cases of Comparative Example is considered to be as follows. Namely, since the curve of the inside shaft 35 in the second case of Specific Example 1 was restrained and the distal end 30a of the outside shaft 30 in Specific Example 1 was closer to the heating member 40 compared to Comparative Example, a larger part of the liquid supplied from the liquid delivery path LP was directed to the heating member 40 and could efficiently diffuse the heated liquid in the vicinity of the heating member 40, compared to the second case of Comparative Example. In addition, since a larger part of the liquid supplied from the liquid delivery path LP was directed to the heating member 40 compared to the second case of Comparative Example, the liquid was efficiently heated by the heating member 40.

### <<Result of Specific Example 2 in First Case>>

In the balloon catheter 15 according to Specific Example 2, in the first case, an alignment direction of the distal end 30a of the outside shaft 30 and the heating member 40 was along the center axis 30X of the outside shaft 30 at the distal end 30a of the outside shaft 30. In this case, by adjusting a temperature of the heating member 40 to 65°C, the balloon 25 had a surface temperature of 65°C. Namely, the balloon 25 could have a surface temperature of 65°C by adjusting the temperature of the heating member 40 to a temperature lower than that of the heating member 40 in Comparative Example. The reason is considered to be as follows. Since the distal end 30a of the outside shaft 30 in Specific Example 2 was closer to the heating member 40 compared to Comparative Example, a larger part of the liquid supplied from the liquid delivery path LP was directed to the heating member 40 and could efficiently diffuse the heated liquid in the vicinity of the heating member 40, compared to the first case of Comparative Example. In addition, since a larger part of the liquid supplied from the liquid delivery path LP was directed to the heating member 40 compared to the first case of Comparative Example, the liquid was efficiently heated by the heating member 40.

### <<Result of Specific Example 2 in Second Case>>

In the second case, although the inside shaft 35 of the catheter 15 according to Specific Example 2 was curved between the distal end 30a of the outside shaft 30 and the heating member 40, such curve was gentle compared to the inside shaft 35 in Comparative Example in the second case. The balloon 25 had a surface temperature of 64°C by adjusting the temperature of the heating member 40 to 65°C. Namely, the surface temperature of the balloon 25 was lower by 1°C only, compared to that of the first case. The reason of a smaller difference between the surface temperatures of the balloon 25 in the first and second cases compared to the first and second cases of Comparative Example is considered to be as follows. Namely, since the curve of the inside shaft 35 in the second case of Specific Example 2 was restrained and the distal end 30a of the outside shaft 30 in Specific Example 2 was closer to the heating member 40 compared to Comparative Example, a larger part of the liquid supplied from the liquid delivery path LP was directed to the heating member 40 and could efficiently diffuse the heated liquid in the vicinity of the heating member 40, compared to the second case of Comparative Example. In addition, since a larger part of the liquid supplied from the liquid delivery path LP was directed to the heating member 40 compared to the second case of Comparative Example, the liquid was efficiently heated by the heating member 40.

### <<Result of Specific Example 3 in First Case>>

In the balloon catheter 15 according to Specific Example 3, in the first case, an alignment direction of the distal end 30a of the outside shaft 30 and the heating member 40 was along the center axis 30X of the outside shaft 30 at the distal end 30a of the outside shaft 30. In this case, by adjusting a temperature of the heating member 40 to 65°C, the balloon 25 had a surface temperature of 65°C. Namely, the balloon 25 could have a surface temperature of 65°C by adjusting the temperature of the heating member 40 to a temperature lower than that of the heating member 40 in Comparative Example. The reason is considered to be as follows. Since the distal end 30a of the outside shaft 30 in Specific Example 2 was closer to the heating member 40 compared to Comparative Example, a larger part of the liquid supplied from the liquid delivery path LP was directed to the heating member 40 and could efficiently diffuse the heated liquid in the vicinity of the heating member 40, compared to the first case of Comparative Example. In addition, since a larger part of the liquid supplied from the liquid delivery path LP was directed to the heating member 40 compared to the first case of Comparative Example, the liquid was efficiently heated by the heating member 40.

### <<Result of Specific Example 3 in Second Case>>

In the second case, although the inside shaft 35 of the catheter 15 according to Specific Example 1 was curved between the distal end 30a of the outside shaft 30 and the heating member 40, such curve was gentle compared to the inside shaft 35 in Comparative Example in the second case. Further, such curve was even gentler compared to Specific Examples 1 and 2 in the second case. The balloon 25 had a surface temperature of 65°C by adjusting the temperature of the heating member 40 to 65°C. Namely, the surface temperature of the balloon 25 was the same as that of the first case of Specific Example 3. The reason of a smaller difference between the surface temperatures of the balloon 25 in the first and second cases even when compared to Specific Examples 1 and 2 is considered to be as follows. Namely, since the curve of the inside shaft 35 in Specific Example 3 in the second case was more restrained compared to the second cases of Specific Examples 1 and 2, a larger part of the liquid supplied from the liquid delivery path LP was directed to the heating member 40 and could more efficiently diffuse the heated liquid in the vicinity of the heating member 40, compared to the second cases of Specific Examples 1 and 2. In addition, since a larger part of the liquid supplied from the liquid delivery path LP was directed to the heating member 40 compared to the second cases of Specific Examples 1 and 2, the liquid was more efficiently heated by the heating member 40.

The balloon catheter 15 and the balloon catheter system 10 according to the above embodiments have been described with reference to Figs. 1 to 10 and 11 to 13, but the structures of the balloon catheter 15 and the balloon catheter system 10 are not limited to the above. The structures of the balloon catheter 15 and the balloon catheter system 10 may be variously modified.

For example, a liquid delivery path through which a liquid discharged from the inner space of the balloon 25 flows may be provided independently of the liquid delivery path LP through which a liquid to be supplied to the inner space of the balloon 25 flows. For example, a lumen as an inner space of the inner shaft 35 may be used as a liquid delivery path through which a liquid discharged from the inner space of the balloon 25 flows.

When the liquid supply path LP through which the liquid to be supplied to the inner space of the balloon 25 flows and the liquid discharge path through which the liquid discharged from the inner space of the balloon 25 flows are separated from each other as described above, the catheter system 10 may have, in addition to the supply device 74 for supplying a liquid to the liquid delivery path LP for supply, a discharge device for discharging a liquid from the liquid discharge path, and such discharge device may be equipped separately from the supply device 74.

In addition, when the liquid supply path LP through which the liquid to be supplied to the inner space of the balloon 25 flows and the liquid discharge path through which the liquid discharged from the inner space of the balloon 25 flows are separated from each other, the agitation device 75 may perform supply of liquid to the liquid supply path LP and discharge of liquid from the liquid discharge path. In this case, since the liquid circulates in a flow path constituted by the liquid supply path LP, the inner space of the balloon 25, and the liquid discharge path, the liquid in the balloon 25 is agitated.

In the aforementioned first to third embodiments, the balloon catheter 15 comprises: the balloon 25; the outside shaft 30 connected to the proximal end 25b of the balloon 25; the inside shaft 35 extending inside the outside shaft 30 and into the balloon 25 to be connected to the distal end 25a of the balloon 25; and the heating member 40 disposed in the balloon 25 for heating a liquid in the balloon 25. A distance between the distal end 30a of the outside shaft 30 and the proximal end 40b of the heating member 40 is between 0 mm and 5 mm.

Such a balloon catheter system 15 can restrain the inside shaft 35 from being curved between the distal end 30a of the outside shaft 30 and the heating member 40, because the distal end 30a of the outside shaft 30 and the heating member 40 are close to each other. Thus, even when a force is applied to the balloon 25 or the inside shaft 35 in a direction intersecting the direction along the center axis 30X at the distal end 30a of the outside shaft 30, much of the liquid supplied from the liquid delivery path LP into the balloon 25 can be directed to the heating member 40 in a reliable manner. In addition, the heated liquid in the vicinity of the heating member 40 can be efficiently diffused in a reliable manner. Moreover, the liquid supplied from the liquid delivery path LP into the balloon 25 can be efficiently heated by the heating member 40 in a reliable manner.

Further, since the distal end 30a of the outside shaft 30 and the heating member 40 are close to each other, much more of the liquid supplied from the liquid delivery path LP into the balloon 25 can be directed to the heating member 40. Namely, not only when a force is applied to the balloon 25 or the inside shaft 35 in a direction intersecting the direction along the center axis 30X at the distal end 30a of the outside shaft 30, but also when a force is applied to the aforementioned direction along the center axis 30X, much more of the liquid can be directed to the heating member 40. Thus, diffusion efficiency of the heated liquid in the vicinity of the heating member 40 can be improved, and heating efficiency of the liquid supplied from the liquid delivery path LP into the balloon 25 can also be improved.

Specifically, in the second embodiment, since the midpoint of the heating member 40 in the direction of the center axis 35X of the inside shaft 35 is positioned closer to the proximal end 25b of the balloon 25 than the midpoint between the proximal end 25b of the balloon 25 and the distal end 25a thereof, the distal end 30a of the outside shaft 30 and the heating member 40 are close to each other.

In the first and third embodiments, since the outside shaft 30 extends into the balloon 25, the distal end 30a of the outside shaft 30 and the heating member 40 are close to each other.

In the aforementioned first and third embodiments, the balloon catheter 15 comprises: the balloon 25; the outside shaft 30 connected to the proximal end 25b of the balloon 25; the inside shaft 35 extending inside the outside shaft 30 and into the balloon 25 to be connected to the distal end 25a of the balloon 25; and the heating member 40 disposed in the balloon 25 for heating a liquid in the balloon 25. The outside shaft 30 extends into the balloon 25.

Such a balloon catheter 15 can allow the distal end 30a of the outside shaft 30 and the heating member 40 to be close to each other, and can restrain the inside shaft 35 from being curved between the distal end 30a of the outside shaft 30 and the heating member 40. As a result, the heated liquid in the vicinity of the heating member 40 can be efficiently diffused in a reliable manner. The liquid supplied from the liquid delivery path LP into the balloon 25 can also be efficiently heated by the heating member 40 in a reliable manner. In addition, since the distal end 30a of the outside shaft 30 and the heating member 40 are close to each other, diffusion efficiency of the heated liquid in the vicinity of the heating member 40 can be improved, and heating efficiency of the liquid supplied from the liquid delivery path LP into the balloon 25 can also be improved.

In the aforementioned first and third embodiments, the through hole 31 is made in a part of the outside shaft 30, the part extending into the balloon 25. In this case, even when the distal end 30a of the outside shaft 30 is immersed in the liquid in the balloon 25 during supply of liquid into the balloon 25 to inflate the balloon 25 and venting of air from inside the balloon 25 , air can be vented through the through hole 31.

In the aforementioned third embodiment, the balloon catheter 15 further comprises the reinforcement tube 36 covering the outer circumferential surface of the inside shaft 35. The reinforcement tube 36 extends from between the proximal end 40b of the heating member 40 and the distal end 30a of the outside shaft 30, into the outside shaft 30. Such a reinforcement tube 36 can further effectively restrain the inside shaft 35 from being curved between the distal end 30a of the outside shaft 30 and the heating member 40.

In the aforementioned third embodiment, a distance between the proximal end 40b of the heating member 40 and the distal end 36a of the reinforcement tube 36 is between 0 mm and 5 mm. Such positioning of the reinforcement tube 36 allows the reinforcement tube 36 to effectively restrain the curving of the inside shaft 35.

In the aforementioned first to third embodiments, the balloon catheter 15 further comprises the temperature sensor 45 disposed in the inner space of the balloon 25. Thus, information on a temperature of the liquid in the balloon 25 can be acquired, whereby the temperature of the liquid in the balloon 25 and thus a surface temperature of the balloon 25 can be properly controlled.

In the aforementioned first to third embodiments, the balloon catheter system 10 comprises: the aforementioned balloon catheter 15; the supply device 74 that supplies a liquid to the liquid delivery path LP formed between the outside shaft 30 and the inside shaft 35; the agitation device 75 that agitates the liquid in the balloon 25 by repeating supply of liquid to the liquid delivery path LP and discharge of liquid from the liquid delivery path LP; and the heating device 70 electrically connected to the heating member 40 to apply electric energy to the heating member 40.

Such a balloon catheter system 10 allows the heated liquid in the vicinity of the heating member 40 to be efficiently diffused in a reliable manner. In addition, the liquid supplied from the liquid delivery path LP into the balloon 25 can be efficiently heated by the heating member 40 in a reliable manner. Thus, a surface temperature of the balloon 25 can be controlled to an ideal temperature in a reliable manner.

The present invention can be used for a balloon catheter system and a balloon catheter for treating arrhythmias such as atrial fibrillation, endometriosis, cancer, etc.

- 10: Balloon catheter system
- 15: Balloon catheter
- 25: Balloon
- 25a: Distal end
- 25b: Proximal end
- 30: Outside shaft
- 30a: Distal end
- 30b: Proximal end
- 31: Through hole
- 35: Inside shaft
- 36: Reinforcement tube
- 36a: Distal end
- 36b: Proximal end
- 40: Heating member
- 40b: Proximal end
- 41: Electrode for high-frequency current conduction
- 42: Lead wire for high-frequency current conduction
- 45: Temperature sensor
- 46: Thermosensitive part
- 47: Lead wire for temperature sensor
- 70: Heating device
- 75: Agitation device
- LD: Longitudinal direction
- LP: Liquid delivery path

## Claims

1. A balloon catheter comprising:
a balloon;
an outside shaft connected to a proximal end of the balloon;
an inside shaft extending inside the outside shaft and into the balloon to be connected to a distal end of the balloon; and
a heating member disposed in the balloon for heating a liquid in the balloon,
wherein a distance between a distal end of the outside shaft and a proximal end of the heating member is between 0 mm and 5 mm.

2. The balloon catheter according to claim 1, wherein
a midpoint of the heating member in a direction of a center axis of the inside shaft is positioned closer to the proximal end of the balloon than a midpoint between the proximal end of the balloon and the distal end thereof.

3. The balloon catheter according to claim 1 or 2, wherein
the outside shaft extends into the balloon.

4. A balloon catheter comprising:
a balloon;
an outside shaft connected to a proximal end of the balloon;
an inside shaft extending inside the outside shaft and into the balloon to be connected to a distal end of the balloon; and
a heating member disposed in the balloon for heating a liquid in the balloon;
wherein the outside shaft extends into the balloon.

5. The balloon catheter according to claim 3 or 4, wherein
a through hole is provided in a part of the outside shaft, the part extending into the balloon.

6. The balloon catheter according to any one of claims 1 to 5, comprising a reinforcement tube covering an outer circumferential surface of the inside shaft,
wherein the reinforcement tube extends from between a proximal end of the heating member and a distal end of the outside shaft, into the outside shaft.

7. The balloon catheter according to claim 6, wherein
a distance between the proximal end of the heating member and a distal end of the reinforcement tube is between 0 mm and 5 mm.

8. The balloon catheter according to any one of claims 1 to 7, comprising a temperature sensor disposed in an inner space of the balloon.

9. A balloon catheter system comprising:
a balloon catheter according to any one of claims 1 to 8;
a supply device that supplies a liquid into a liquid delivery path formed between the outside shaft and the inside shaft;
an agitation device that agitates a liquid in the balloon by repeating supply of liquid to the liquid delivery path and discharge of liquid from the liquid delivery path; and
a heating device electrically connected to the heating member to apply electric energy to the heating member.
